# EUROPÄISCHE PATENTSCHRIFT

(11) **EP 1 844 704 B1**
(45) Veröffentlichungstag und Bekanntmachung des Hinweises auf die Patenterteilung: **23.09.2009**
(21) Anmeldenummer: 07003204.0
(22) Anmeldetag: 15.02.2007
(51) Int. Cl.: A61B 3/103

(54) **Refraktionsmessvorrichtung zur Bestimmung der Refraktionseigenschaften eines Auges**
Refraction measuring device for determining the refraction characteristics of an eye
Dispositif de mesure de réfraction destiné à la détermination des propriétés de réfraction de l'oeil

(30) Priorität: 11.04.2006 DE 102006017389
(43) Veröffentlichungstag der Anmeldung: 17.10.2007
(73) Patentinhaber: Oculus Optikgeräte GmbH, 35582 Wetzlar-Dutenhofen (DE)
(72) Erfinder: Köst, Gert, 30171 Hannover (DE)
(74) Vertreter: von den Steinen, Axel

(56) Entgegenhaltungen:
- EP-A- 0 563 454
- EP-A- 0 962 184
- EP-A- 1 308 128
- EP-A1- 1 074 214
- WO-A-02/080760
- WO-A-20/04037078
- DE-A1- 19 719 694
- DE-C- 456 169
- DE-U1- 29 913 602
- US-A- 5 886 767
- US-A- 6 033 071
- US-B1- 6 309 068

## Beschreibung

Die Erfindung betrifft eine Refraktionsmessvorrichtung zur Bestimmung der Refraktionseigenschaften eines Auges nach dem Oberbegriff des Anspruchs 1.

Gattungsgemäße Refraktionsmessvorrichtungen werden vielfach auch als Autorefraktometer bezeichnet und dienen dazu, die Refraktion eines Auges und gegebenenfalls eine Fehlsichtigkeit des Auges aus der Distanz zu messen.

Das Grundprinzip gattungsgemäßer Refraktionsmessvorrichtungen ist in der US 4,761,070 beschrieben. Mit Hilfe einer optischen Projektionseinrichtung, beispielsweise Leuchtdioden, wird ein Beleuchtungsmuster erzeugt und auf die Netzhaut des Auges projiziert. Die Projektion des Beleuchtungsmusters erfolgt dabei insbesondere so, dass das Beleuchtungsmuster auf der Netzhaut fokussiert ist. Mit einer optischen Beobachtungseinrichtung, die einen photoelektrischen Sensor, beispielsweise eine Videokamera, umfasst, wird das an der Netzhaut reflektierte Beleuchtungsmuster durch die Augenlinse hindurch betrachtet, so dass ein Abbildungsmuster auf dem photoelektrischen Sensor abgebildet wird. Dieses Abbildungsmuster wird mit dem photoelektrischen Sensor aufgenommen und mit einer Auswerteeinrichtung, vorzugsweise einer digitalen Bilddatenverarbeitungssoftware, ausgewertet. Entsprechend der

Refraktionseigenschaften des Auges wird das auf die Netzhaut projizierte Beleuchtungsmuster charakteristisch verzerrt, so dass durch die Auswertung des Maß der Verzerrung die Refraktionseigenschaften des Auges abgeleitet werden können.

Von großer Bedeutung für die zutreffende Ableitung der Refraktionseigenschaften ist es, dass das Auge in einem bestimmten Abstand zur Refraktionsmessvorrichtung angeordnet ist. Denn Abstandsabweichungen zwischen Auge und Refraktionsmessvorrichtung führen dazu, dass das Beleuchtungsmuster entsprechend anders verzerrt auf den photoelektrischen Sensor abgebildet wird, so dass die Abstandsabweichungen zu Messfehlern bei der Refraktionsbestimmung führen.

Zur Vermeidung dieser Messfehler beschreibt die DE 101 53 397 A1 ein Refraktionsmesssystem, das zusätzlich eine Messeinheit zur Bestimmung des Abstandes zwischen Gerät und Patient aufweist. Die durch die Distanzmessung gewonnenen Daten können dazu verwendet werden, den Patienten ordnungsgemäß vor dem Gerät zu positionieren. Zusätzlich ist es danach auch denkbar, die Abstandsinformationen in die Auswertung des Refraktionsmessverfahrens einfließen zu lassen, um die Refraktionsmessdaten entsprechend zu korrigieren.

Als mögliche Messsysteme zur Bestimmung des Abstandes beschreibt die DE 101 53 397 A1 einen Ultraschallsender und -empfänger beziehungsweise ein optisches Distanzmesssystem, bei dem ein Lichtmuster auf die Stirn des Probanden projiziert wird, um auf diese Weise den Abstand zwischen Stirn und Messsystem auszumessen.

Nachteilig an der in der DE 101 53 397 A1 beschriebenen Messvorrichtung ist es, dass mit der dort beschriebenen Abstandsmessung nur der Abstand zwischen dem Kopf des Patienten und der Refraktionsmesseinrichtung gemessen werden kann. Diese Messung ist deshalb ungenau und im Prinzip ungenügend, um die Refraktionsmessdaten geeignet zu korrigieren, da die Position des Kopfes keine eindeutige Aussage über die Position des Auges enthält. Die Messdatenkorrektur führt deshalb zu ungenügenden Ergebnissen.

Aus der EP 0 563 454 A1 ist ein Augenrefraktometer bekannt, wobei eine in das Refraktometer integrierte Positioniervorrichtung, welche ein Interferometer umfasst, eine präzise Bestimmung eines Abstandes eines Auges von einer Optik des Refraktometers ermöglicht.

Eine weitere Refraktionsmessvorrichtung mit einer Abstandsmesseinrichtung zur Messung eines Abstandes zu einem Auge ist aus der US 6,033,071 bekannt. Die Abstandmesseinrichtung ist im Wesentlichen aus einem flexiblen Draht gebildet, welcher zwischen dem Auge und einer Linse positionierbar und in seiner Länge veränderlich ist.

Ein Refraktometer mit der Möglichkeit einer Messung eines Hornhautabstandes zum Refraktometer ist aus der DE 456 169 bekannt. Das Refraktometer umfasst ein Fernrohrobjektiv mit zwei Linsen, wobei eine Linse zur Abstandsmessung vorgesehen ist.

Die US 6,309,068 B1 offenbart ein ophthalmolagisches Gerät, welches sich aus einem Autorefraktometer und einem Autokeratometer zusammensetzt. Das Gerät ermöglicht eine automatische Abstandsmessung zu einem Auge.

Ausgehend von diesem Stand der Technik ist es deshalb Aufgabe der vorliegenden Erfindung, eine neue Refraktionsmessvorrichtung vorzuschlagen, bei der durch eine direkte Messung eines Abstandes zwischen der Refraktionsmessvorrichtung und einem Auge Refraktionsmessdaten mit erhöhter Messgenauigkeit erhalten werden können.

Diese Aufgabe wird durch eine Refraktionsmessvorrichtung nach der Lehre des Anspruchs 1 gelöst.

Vorteilhafte Ausführungsformen der Erfindung sind Gegenstand der Unteransprüche.

Die Erfindung beruht auf dem Grundgedanken, dass die in der Refraktionsmessvorrichtung vorgesehene Abstandsmesseinrichtung dazu geeignet ist, den Abstand zwischen der Refraktionsmessvorrichtung und dem zu untersuchenden Auge zu messen, da es allein auf diesen Abstand für die Messdatenkorrektur ankommt. Erst durch die Verwendung dieser Abstandsdaten zur Beschreibung des Abstandes zwischen Auge und Refraktionsmessvorrichtung gelingt es, den Patienten entsprechend korrekt zu positionieren beziehungsweise die gemessenen Refraktionsmessdaten geeignet zu korrigieren.

In welcher Bauart die Abstandsmesseinrichtung ausgebildet ist, ist grundsätzlich beliebig. Als besonders geeignet und messgenau hat sich eine Abstandsmesseinrichtung erwiesen, die eine Spaltprojektionseinrichtung zur Spaltbeleuchtung des Auges und eine Scheimpflugaufnahmeeinrichtung zur Aufnahme von Schnittbildern des Auges umfasst. Durch Auswertung der Bilddaten der mit der Scheimpflugaufnahmeeinrichtung aufgenommenen Schnittbilder kann der Abstand zwischen der Refraktionsmessvorrichtung und dem Auge außerordentlich genau abgeleitet werden.

Die Messgenauigkeit kann dabei noch erhöht werden, wenn mit der Abstandsmesseinrichtung der Abstand zwischen der Refraktionsmesseinrichtung und der Hornhaut des Auges, insbesondere der Vorderfläche der Hornhaut und/oder der Hinterfläche des Auges, messbar ist.

Alternativ beziehungsweise additiv dazu ist es besonders vorteilhaft, wenn mit der Abstandsmesseinrichtung auch der Abstand zwischen der Refraktionsmessvorrichtung und der Linse des Auges, insbesondere der Vorderfläche der Linse und/oder der Hinterfläche der Linse, messbar ist.

Soweit in der Refraktionsmessvorrichtung eine Scheimpflugkamera zur Abstandsbestimmung eingesetzt wird, kann diese Scheimpflugaufnahmeeinrichtung selbstverständlich auch zur Durchführung konventioneller tachiometrischer Messaufgaben eingesetzt werden. Insbesondere kann dann also auch die Dicke des Hornhautgewebes aus den Schnittbildern des Auges abgeleitet werden.

Für die Durchführung einer korrekten Messung ist es von Vorteil, dass sich das Auge während der Messung nicht bewegt. Deshalb ist es besonders vorteilhaft, wenn in der Refraktionsmessvorrichtung eine Fixationsmarke vorgesehen ist, die während des Messens vom Auge fixiert wird, um dadurch unerwünschte Augenbewegungen zu vermeiden.

Um den Funktionsumfang der Refraktionsmessvorrichtung noch zu erweitern, kann zusätzlich noch eine zweite optische Projektionseinrichtung und eine zweite optische Projektionseinrichtung und eine zweite optische Beobachtungseinrichtung in die Refraktionsmessvorrichtung integriert werden, wobei die zweite optische Projektionseinrichtung zusammen mit der zweiten optische Beobachtungseinrichtung und einer geeigneten Auswerteeinrichtung ein Keratometer bilden. Die Bauart der Messmarken des Keratometers, die Teil der Projektionseinrichtung sind, ist grundsätzlich beliebig. Besonders bevorzugt ist es, wenn zwei kollimierte Leuchtpunkte und ein im Wesentlichen kreisförmiger, nicht kollimierter Leuchtstreifen als Messmarken in der Projektionseinrichtung des Keratometers vorgesehen sind.

Zur Bildung der kollimierten Leuchtpunkte können bevorzugt Leuchtdioden eingesetzt werden, die in einem Tubus angeordnet sind, wobei den Leuchtdioden dabei jeweils Linsen vorgeordnet werden.

Der kreisförmige, nicht kollimierte Leuchtstreifen kann' bevorzugt von einem kreiszylindrischen Lichtleiterelement erzeugt werden. Das Licht wird dabei von einem Leuchtmittel an der hinteren Stirnseite und/oder am Zylinderumfang ins Lichtleiterelement eingekoppelt und tritt an der vorderen Stirnseite aus dem Lichtleitelement aus. Als Leuchtmittel für das kreiszylindrische Lichtleiterelement können wiederum Leuchtdioden eingesetzt werden, die bevorzugt über den Umfang des kreiszylindrischen Lichtleiterelements verteilt sind.

Welche Lichtquelle zur Erzeugung des Beleuchtungsmusters in der optischen Projektionseinrichtung der Refraktionsmesseinrichtung verwendet wird, ist grundsätzlich beliebig. Als besonders vorteilhaft haben sich dabei Infrarotlichtquellen erwiesen.

In der optischen Beobachtungseinrichtung der Refraktionsmessvorrichtung sollte bevorzugt eine Lochblende vorgesehen sein.

Die Bauart der photoelektrischen Sensoren in den verschiedenen Beobachtungseinrichtungen der Refraktionsmessvorrichtung beziehungsweise der Scheimpflugeinrichtung beziehungsweise des Keratometers ist grundsätzlich beliebig. Bevorzugt können dafür Videosensoren eingesetzt werden, die die aufgenommenen Bilddaten in ein Videosignal umwandeln und an nachgeordnete Funktionseinheiten weiterleiten. Besonders preisgünstig können die Videosensoren von Chipkameras, insbesondere CCD-Kammeras gebildet werden.

Das Videosignal des Videosensors sollte dabei bevorzugt ein digitales Datenformat aufweisen, um eine digitale Bilddatenverarbeitung in einfacher Weise zu ermöglichen.

Um das zu untersuchende Auge vor Beginn der Messung einfach ausrichten zu können, beziehungsweise während der Untersuchung beobachten zu können, sollte in der Refraktionsmessvorrichtung eine Einrichtekamera beziehungsweise Übersichtskamera vorgesehen sein. Bei geeigneter Anordnung kann die Einrichtekamera dabei zugleich auch als Übersichtskamera während der Messung dienen.

Soweit in der Refraktionsmessvorrichtung ein Keratometer integriert ist, kann die optische Beobachtungseinrichtung des Keratometers zugleich auch als Einrichtekamera beziehungsweise Übersichtskamera eingesetzt werden.

Eine Ausführungsform der Erfindung ist in der Zeichnung schematisch dargestellt und wird beispielhaft erläutert.

Es zeigt:

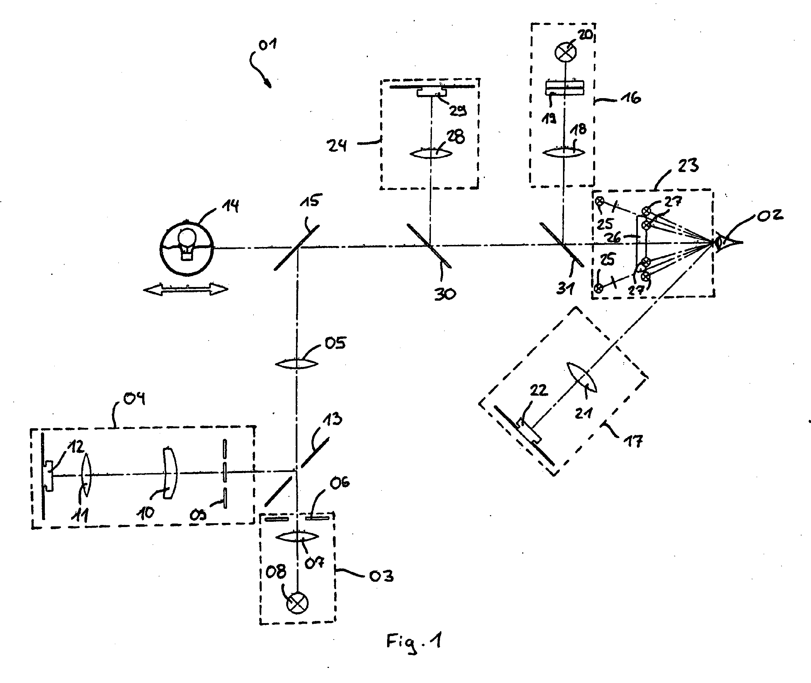

- **Fig. 1:**: Den schematischen Aufbau einer Ausführungsform einer erfindungsgemäßen Refraktionsmessvorrichtung im Überblick.

In **Fig. 1** ist der schematisierte Strahlengang einer Refraktionsmessvorrichtung 01 zur Durchführung von Messungen am Auge 02 schematisch dargestellt.

Zur Bestimmung der Refraktionseigenschaften des Auges 02 dient eine optische Projektionseinrichtung 03 und eine optische Beobachtungseinrichtung 04, die zusammen mit einem Objektiv 05 und einer nicht dargestellten Auswerteeinrichtung einen Autorefraktor bilden. Mit der optischen Projektionseinrichtung 03 kann ein Beleuchtungsmuster auf die Netzhaut des Auges 02 projiziert und dort fokussiert werden. Die optische Projektionseinrichtung 03 umfasst dabei eine Lochblende 06, eine Linse 07 und eine Infrarotlichtquelle 08. Die optische Beobachtungseinrichtung 04 des Autorefraktors umfasst eine 6-fach Lochblende 09, ein Ablenkprisma 10, ein Objektiv 11 und eine CCD-Kamera 12. Die mit der CCD-Kamera 12 aufgenommenen Bilddaten werden in der nachgelagerten Auswerteeinrichtung, die in der Art eines digitalen Bildverarbeitungssystems ausgebildet ist, ausgewertet, um die Refraktionseigenschaften des Auges 02 zu bestimmen. Zur Einkoppelung der verschiedenen Strahlengänge der optischen Projektionseinrichtung 03 und der optischen Beobachtungseinrichtung 04 dient ein Spiegel 13 mit Lochblende.

Um das Auge 02 während der Durchführung der Messung fixieren zu können, umfasst die Refraktionsmessvorrichtung 01 weiterhin eine verstellbar gelagerte Fixationsmarke 14, deren Strahlengang mittels einer Teilerplatte 15 eingespeist wird.

Um den Abstand des Auges 02 relativ zur Refraktionsmessvorrichtung bestimmen zu können, ist eine Abstandsmessvorrichtung vorgesehen, die von einer Spaltprojektionseinrichtung 16 und einer Scheimpflugaufnahmeeinrichtung 17 gebildet ist. Die Spaltprojektionseinrichtung 16 und die Scheimpflugaufnahmeeinrichtung 17 sind dabei entsprechend der Scheimpflugregel angeordnet, so dass die mit der Scheimpflugaufnahmeeinrichtung 17 aufgenommenen Schnittbilder dazu geeignet sind, den Abstand zwischen der Refraktionsmessvorrichtung 01 und dem Auge 02 durch Bilddatenanalyse zu bestimmen. Insbesondere kann durch Bilddatenanalyse der Schnittbilder des Auges 02 auch der Abstand der Refraktionsmessvorrichtung 01 zur Hornhaut des Auges, insbesondere der Vorderfläche beziehungsweise Hinterfläche der Hornhaut und auch der Abstand der Refraktionsmessvorrichtung 01 zur Linse des Auges, besonders der Vorderfläche der Linse beziehungsweise der Hinterfläche der Linse, bestimmt werden. Darüber hinaus kann die Spaltprojektionseinrichtung 16 zusammen mit der Scheimpflugaufnahmeeinrichtung 17 selbstverständlich auch zur Durchführung normaler tachiometrischer Messungen, insbesondere der Messung der Hornhautdicke, eingesetzt werden. Die Spaltprojektionseinrichtung 16 umfasst dabei ein Objektiv 18, eine Spaltblende 19 und eine Spaltlampe 20. Die Scheimpflugaufnahmeeinrichtung 17 ist entsprechend der Scheimpflugregel winkelig relativ zum Auge 02 angeordnet und umfasst ein Objektiv 21 und eine als Scheimpflugaufnahmeeinrichtung dienende CCD-Kamera 22.

Zusätzlich ist in die Refraktionsmessvorrichtung 01 noch ein Keratometer integriert, das aus einer geeigneten Projektionseinrichtung 23 und einer zugeordneten optischen Beobachtungseinrichtung 24 besteht. In der optischen Projektionseinrichtung dienen zwei Leuchtdioden 25 als kollimierte Leuchtpunkte. Ein Lichtleiterelement 26 mit zugeordneten LEDs 27 dient als kollimierter Leuchtstreifen mit kreiszylindrischer Geometrie. In der optischen Beobachtungseinrichtung 24 ist ein Objektiv 28 und eine CCD-Kamera 29 vorgesehen. Die CCD-Kamera 29 dient dabei zugleich auch als Einrichteekamera und Übersichtskamera. Zwei Teilerplatten 30 und 31 dienen der Einkopplung des Strahlengangs der Spaltprojektionseinrichtung 16 und der optischen Beobachtungseinrichtung 24 des Keratometers.

## Patentansprüche

1. Refraktionsmessvorrichtung (01) zur Bestimmung der Refraktionseigenschaften eines Auges (02) eines Patienten,
- mit einer optischen Projektionseinrichtung(03), umfassend zumindest eine Lichtquelle (08), die ein Beleuchtungsmuster erzeugen kann, wobei das Beleuchtungsmuster der Projektionseinrichtung (03) auf die Netzhaut des Auges (02) projiziert und dort insbesondere fokussiert werden kann,
- mit einer optischen Beobachtungseinrichtung (04), umfassend zumindest einen photoelektrischen Sensor (12), wobei mit der Beobachtungseinrichtung (04) das an der Netzhaut des Auges (02) reflektierte Beleuchtungsmuster durch die Hornhaut und die Augenlinse hindurch betrachtet und als Abbildungsmuster auf dem photoelektrischen Sensor (12) abgebildet werden kann,
- mit einer Auswerteeinrichtung, mit der das vom photoelektrischen Sensor (12) aufgenommene Abbildungsmuster unter Ableitung der Refraktionseigenschaften des Auges (02) ausgewertet werden kann,
- mit einer Abstandsmesscinrichtung (16, 17) zur Bestimmung des Abstandes zwischen der Refraktionsmessvorrichtung (01) und dem Patient,
**dadurch gekennzeichnet,**
**dass** mit der Abstandsmesseinrichtung (16, 17) der Abstand zwischen der Refraktionsmessvorrichtung (01) und dem Auge (02) messbar ist, wobei die Abstandsmesseinrichtung (16, 17) eine Spaltprojcktionseinrichtung (16) zur Spaltbeleuchtung des Auges (02) und einer Scheimpflugaufnahmeeinrichtung (17) zur Aufnahme von Schnittbildern des Auges (02) umfasst, wobei der Abstand zwischen der Refraktionsmessvorrichtung (01) und dem Auge (02) mit einer Auswerteeinrichtung aus den Schnittbildern des Auges (02) abgeleitet werden kann.

2. Refraktionsmessvorrichtung nach Anspruch 1,
**dadurch gekennzeichnet,**
**dass** der Abstandsmesseinrichtung (16, 17) der Abstand zwischen der Refraktionsmessvorrichtung (01) und der Hornhaut des Auges (02), insbesondere der Vorderfläche der Hornhaut und/oder der Hinterfläche der Hornhaut, messbar ist.

3. Refraktionsmessvorrichtung nach Anspruch 1 oder 2,
**dadurch gekennzeichnet,**
**dass** der Abstandsmesseinrichtung (16, 17) der Abstand zwischen der Refraktionsmessvorrichtung (01) und der Linse des Auges (01), insbesondere der Vorderfläche der Linse und/oder Hinterfläche der Linse, messbar ist.

4. Refraktionsmessvorrichtung nach einem der Ansprüche 1 bis 3,
**dadurch gekennzeichnet,**
**dass** in einer Auswerteeinrichtung aus den Schnittbildern des Auges (02) auch die Dicke des Hornhautgewebes abgeleitet werden kann.

5. Refraktionsmessvorrichtung nach einem der Ansprüche 1 bis 4,
**dadurch gekennzeichnet,**
**dass** in der Refraktionsmessvorrichtung (01) eine Fixationsmarke (14) vorgesehen ist, die vom Auge (02) während einer Messung fixiert wird.

6. Refraktionsmessvorrichtung nach Anspruch 5,
**dadurch gekennzeichnet,**
**dass** der tatsächliche oder virtuelle Abstand zwischen Auge (02) und Fixationsmarke (14) veränderbar ist.

7. Refraktionsmessvorrichtung nach Anspruch 6,
**dadurch gekennzeichnet,**
**dass** der tatsächliche Abstand zwischen Auge und Fixationsmarke durch Verstellung der Fixationsmarke in der Refraktionsmessvorrichtung variiert werden kann.

8. Refraktionsmessvorrichtung nach Anspruch 6,
**dadurch gekennzeichnet,**
**dass** der virtuelle Abstand zwischen Auge und Fixationsmarke durch Verstellung zumindest einer Linse im Strahlengang zwischen Fixationsmarke und Auge variiert werden kann.

9. Refraktionsmessvorrichtung nach einem der Ansprüche 1 bis 8,
**dadurch gekennzeichnet,**
**dass** die Refraktionsmessvorrichtung (01) eine zweite optische Projektionseinrichtung (23) und eine zweite optischen Beobachtungseinrichtung (24) die zusammen mit einer Auswerteeinrichtung ein Keratometer bilden.

10. Refraktionsmessvorrichtung nach Anspruch 9,
**dadurch gekennzeichnet,**
**dass** mit der Projektionseinrichtung (23) des Keratometers eine definierte Messmarke auf die Hornhaut projizierbar ist.

11. Refraktionsmessvorrichtung nach Anspruch 9 oder 10,
**dadurch gekennzeichnet,**
**dass** die Messmarke zwei kollimierte Leuchtpunkte und einen im wesentlichen kreisförmigen, nicht kollimierte Leuchtstreifen aufweist.

12. Refraktionsmessvorrichtung nach Anspruch 11,
**dadurch gekennzeichnet,**
**dass** die kollimierte Leuchtpunkte jeweils von einer in einem Tubus angeordneten Leuchtdiode (25), der zumindest eine Linse vorgcordnet ist, erzeugt werden.

13. Refraktionsmessvorrichtung nach Anspruch 11 oder 12,
**dadurch gekennzeichnet,**
**dass** der kreisförmige, nicht kollimierte Leuchtstreifen von einem kreiszylindrischen Lichtleiterelement (26) erzeugt wird, wobei das Licht von zumindest einem Leuchtmittel (27) an der hinteren Stirnseite und/oder am Zylinderumfang ins Lichtleiterelement (26) eingekoppelt wird und an der vorderen Stirnseite aus dem Lichtleiterelement austritt.

14. Analysesystem nach Anspruch 13,
**dadurch gekennzeichnet,**
**dass** als Leuchtmittel mehrere über den Umfang des kreiszylindrischen Lichtleiterelements (26) verteilte Leuchtdioden (27) dienen.

15. Refraktionsmessvorrichtung nach einem der Ansprüche 1 bis 14,
**dadurch gekennzeichnet,**
**dass** in der optischen Projektionseinrichtung (03) der Refraktionsmessvorrichtung (01) eine Infrarotlichtquelle (08) zur Erzeugung des Beleuchtungsmusters vorgesehen ist.

16. Refraktionsmessvorrichtung nach einem der Ansprüche 1 bis 15,
**dadurch gekennzeichnet,**
**dass** in der optischen Beobachtungseinrichtung (04) der Refraktionsmessvorrichtung (01) eine Lochblende (09) vorgesehen ist.

17. Refraktionsmessvorrichtung nach einem der Ansprüche 1 bis 16,
**dadurch gekennzeichnet,**
**dass** in der optischen Beobachtungseinrichtung (04) der Refraktionsmessvorrichtung (01) und/oder in der Scheimpflugaufnahmeeinrichtung (17) und/oder in der optischen Beobachtungseinrichtung (24) des Keratometers jeweils zumindest ein Videosensor (12, 22, 29) vorgesehen ist, wobei der Videosensor (12, 22, 29) die Bilddaten in Form eines Videosignal weitergibt.

18. Refraktionsmessvorrichtung nach Anspruch 17,
**dadurch gekennzeichnet,**
**dass** die Videosensoren (12, 22, 29) in der Art einer Chipkamera ausgebildet sind.

19. Refraktionsmessvorrichtung nach Anspruch 17 oder 18,
**dadurch gekennzeichnet,**
**dass** das Videosignal in einem digitalem Datenformat erzeugt oder in ein digitales Datenformat umgewandelt wird.

20. Refraktionsmessvorrichtung nach einem der Ansprüche 1 bis 19,
**dadurch gekennzeichnet,**
**dass** als Auswerteeinrichtung der Refraktionsmessvorrichtung (01) und/oder der Scheimpflugaufnahmeeinrichtung (37) und/oder der Beobachtungseinrichtung (24) des Keratometers ein digitales Bildverarbeitungssystem vorgesehen ist, mit dem digitalen Bilddaten auswertbar sind.

21. Refraktionsmessvorrichtung nach einem der Ansprüche 1 bis 20,
**dadurch gekennzeichnet,**
**dass** die Refraktionsmessvorrichtung (01) eine Einrichtekamera zur lagerichtigen Ausrichtung des zu untersuchenden Auges (02) und/oder eine Übersichtskamera zur Beobachtung des zu untersuchenden Auges (02) während der Untersuchung aufweist.

22. Refraktionsmessvorrichtung nach Anspruch 21,
**dadurch gekennzeichnet,**
**dass** die optischen Beobachtungseinrichtung (29) des Keratometers auch als Einrichtekamera und/oder als Übersichtskamera einsetzbar ist.

## Claims

1. A refraction measuring device (01) for determining the refraction properties of an eye (02) of a patient,
- comprising an optical projection device (03), comprising at least one light source (08) which can produce an illumination pattern, wherein the illumination pattern of the projection device (03) can be projected onto the retina of the eye (02) and in particular can be focussed there,
- comprising an optical observation device (04), comprising at least one photoelectric sensor (12), wherein the illumination pattern reflected at the retina of the eye (02) is observed through the cornea and the crystalline lens with the observation device (04) and can be imaged as an imaging pattern on the photoelectric sensor (12),
- comprising an evaluation device with which the imaging pattern recorded by the photoelectric sensor (12) can be evaluated whilst deriving the refraction properties of the eye (2),
- comprising a distance measuring device (16, 17) for determining the distance between the refraction measuring device (01) and the patient,
**characterised in that** the distance between the refraction measuring device (01) and the eye (02) can be measured by means of the distance measuring device (16, 17), wherein the distance measuring device (16, 17) comprises a slit projection device (16) for the slit illumination of the eye (02) and a Scheimpflug recording device (17) for recording sections images of the eye (02), wherein the distance between the refraction measuring device (01) and the eye (02) can be deduced by means of an evaluation device from sectional images of the eye (02).

2. The refraction measuring device according to claim 1, **characterised in that** the distance measuring device (16, 17) can measure the distance between the refraction measuring device (01) and the cornea of the eye (02), in particular the anterior surface of the cornea and/or the posterior surface of the cornea.

3. The refraction measuring device according to claim 1 or 2, **characterised in that** the distance measuring device (16, 17) can measure the distance between the refraction measuring device (01) and the lens of the eye (01), in particular the anterior surface of the lens and/or the posterior surface of the lens.

4. The refraction measuring device according to any one of claims 1 to 3, **characterised in that** the thickness of the corneal tissue can be deduced in an evaluation device from the sectional images of the eye (02).

5. The refraction measuring device according to any one of claims 1 to 4, **characterised in that** a fixation mark (14) is provided in the refraction measuring device (01), which is fixed by the eye (02) during a measurement.

6. The refraction measuring device according to claim 5, **characterised in that** the actual or virtual distance between eye (02) and fixation mark (14) is variable.

7. The refraction measuring device according to claim 6, **characterised in that** the actual distance between eye and fixation mark can be varied by adjusting the fixation mark in the refraction measuring device.

8. The refraction measuring device according to claim 6, **characterised in that** the virtual distance between eye and fixation mark can be varied by adjusting at least one lens in the beam path between fixation mark and eye.

9. The refraction measuring device according to any one of claims 1 to 8, **characterised in that** the refraction measuring device (01) ... a second optical projection device (23) and a second optical observation device (24) which together with an evaluation device form a keratometer.

10. The refraction measuring device according to claim 9, **characterised in that** a defined measurement mark can be projected onto the cornea by means of the projection device (23) of the keratometer.

11. The refraction measuring device according to claim 9 or 10, **characterised in that** the measurement mark comprises two collimated luminous points and a substantially circular, non-collimated luminous strip.

12. The refraction measuring device according to claim 11, **characterised in that** the collimated luminous points are each produced by a light-emitting diode (25) disposed in a tube, having at least one lens in front thereof.

13. The refraction measuring device according to claim 11 or 12, **characterised in that** the circular, non-collimated luminous strip is produced by a circular cylindrical fibre-optic element (26), wherein the light is coupled into the fibre-optic element (26) from at least one illuminant (27) at the rear face and/or on the cylinder circumference and emerges from the fibre-optic element at the front face.

14. The analysis system according to claim 13, **characterised in that** a plurality of light-emitting diodes (27) distributed over the circumference of the circular cylindrical fibre-optic element (26) serve as illuminant.

15. The refraction measuring device according to any one of claims 1 to 14, **characterised in that** an infrared light source (08) for producing the illumination pattern is provided in the optical projection device (03) of the refraction measuring device (01).

16. The refraction measuring device according to any one of claims 1 to 15, **characterised in that** a perforated screen (09) is provided in the optical observation device (04) of the refraction measuring device (01).

17. The refraction measuring device according to any one of claims 1 to 16, **characterised in that** respectively at least one video sensor (12, 22, 29) is provided in the optical observation device (04) of the refraction measuring device (01) and/or in the Scheimpflug recording device (17) and/or in the optical observation device (24) of the keratometer, wherein the video sensor (12, 22, 29) reproduces the image data in the form of a video signal.

18. The refraction measuring device according to claim 17, **characterised in that** the video sensors (12, 22, 29) are configured in the manner of a chip camera.

19. The refraction measuring device according to claim 17 or 18, **characterised in that** the video signal is generated in a digital data format or is converted into a digital data format.

20. The refraction measuring device according to any one of claims 1 to 19, **characterised in that** a digital image processing system with which digital image data can be evaluated is provided as an evaluation device of the refraction measuring device (01) and/or of the Scheimpflug recording device (17) and/or of the observation device (24) of the keratometer.

21. The refraction measuring device according to any one of claims 1 to 20, **characterised in that** the refraction measuring device (01) comprises a setting-up camera for positionally correct alignment of the eye (02) to be studied and/or an overview camera for observing the eye (02) to be studied during the examination.

22. The refraction measuring device according to claim 21, **characterised in that** the optical observation device (29) of the keratometer can also be used as a setting-up camera and/or as an overview camera.

## Revendications

1. Dispositif de mesure de réfraction (01) pour la détermination des propriétés de réfraction d'un oeil (02) d'un patient,
- comportant un dispositif optique de projection (03), comprenant au moins une source lumineuse (08) qui peut générer un modèle d'éclairage, le modèle d'éclairage du dispositif de projection (03) étant projeté sur la rétine de l'oeil (02) et pouvant y être notamment focalisé,
- un dispositif optique d'observation (04), comprenant au moins un capteur photoélectrique (12), le modèle d'éclairage reflété au niveau de la rétine de l'oeil (2) pouvant être observé par la cornée et à travers le cristallin à l'aide du dispositif d'observation (04) et être reproduit sous forme de modèle d'illustration sur le capteur photoélectrique (12),
- un dispositif d'exploitation permettant d'évaluer le modèle d'illustration pris par le capteur photoélectrique (12) en dérivant les propriétés de réfraction de l'oeil (02),
- un dispositif de mesure de distance (16, 17) pour déterminer la distance entre le dispositif de mesure de réfraction (01) et le patient,
**caractérisé en ce que**
le dispositif de mesure de distance (16, 17) permet de mesurer la distance entre le dispositif de mesure de réfraction (01) et l'oeil (02), le dispositif de mesure de distance (16, 17) comprenant un dispositif de projection à fente (16) pour éclairer l'oeil (02) en fente et un dispositif d'enregistrement Scheimpflug (17) pour prendre des images en coupe de l'oeil (02), la distance entre le dispositif de mesure de réfraction (01) et l'oeil (02) pouvant être dérivée des images en coupe de l'oeil (02) à l'aide d'un dispositif d'exploitation.

2. Dispositif de mesure de réfraction selon la revendication 1,
**caractérisé en ce que**
le dispositif de mesure de distance (16, 17) permet de mesurer la distance entre le dispositif de mesure de réfraction (01) et la cornée de l'oeil (02), notamment la surface frontale de la cornée et/ou la surface postérieure de la cornée.

3. Dispositif de mesure de réfraction selon la revendication 1 ou 2,
**caractérisé en ce que**
le dispositif de mesure de distance (16, 17) permet de mesurer la distance entre le dispositif de mesure de réfraction (01) et le cristallin de l'oeil (01), notamment la surface frontale du cristallin et/ou la surface postérieure du cristallin.

4. Dispositif de mesure de réfraction selon une des revendications 1 à 3,
**caractérisé en ce que**,
dans un dispositif d'exploitation, l'épaisseur du tissu de la cornée peut aussi être dérivée des images en coupe de l'oeil (02).

5. Dispositif de mesure de réfraction selon une des revendications 1 à 4,
**caractérisé en ce que**,
dans le dispositif de mesure de réfraction (01), il est prévu une marque de fixation (14) qui est fixée par l'oeil (02) pendant une mesure.

6. Dispositif de mesure de réfraction selon la revendication 5,
**caractérisé en ce que**
la distance réelle ou virtuelle entre l'oeil (02) et la marque de fixation (4) est modifiable.

7. Dispositif de mesure de réfraction selon la revendication 6,
**caractérisé en ce que**
la distance réelle entre l'oeil et la marque de fixation peut être variée en déplaçant la marque de fixation dans le dispositif de mesure de réfraction.

8. Dispositif de mesure de réfraction selon la revendication 6,
**caractérisé en ce que**
la distance virtuelle entre l'oeil et la marque de fixation peut être variée en déplaçant au moins une lentille dans le parcours optique entre la marque de fixation et l'oeil.

9. Dispositif de mesure de réfraction selon une des revendications 1 à 8,
**caractérisé en ce que** le dispositif de mesure de réfraction (01 présente un deuxième dispositif optique de projection (23) et un deuxième dispositif optique d'observation (24) formant avec un dispositif d'exploitation un kératomètre.

10. Dispositif de mesure de réfraction selon la revendication 9,
**caractérisé en ce que**,
avec le dispositif de projection (23) du kératomètre, on peut projeter une marque de mesure définie sur la cornée.

11. Dispositif de mesure de réfraction selon la revendication 9 ou 10,
**caractérisé en ce que**
la marque de mesure présente deux points d'éclairage collimatés et un bandeau d'éclairage sensiblement circulaire et non collimaté.

12. Dispositif de mesure de réfraction selon la revendication 11,
**caractérisé en ce que** les points d'éclairage collimatés sont respectivement générés par une diode électroluminescente (25) disposée dans un tube et en amont de laquelle une lentille est installée.

13. Dispositif de mesure de réfraction selon la revendication 11 ou 12,
**caractérisé en ce que** le bandeau d'éclairage circulaire et non collimaté est généré par un élément conducteur de lumière cylindrique circulaire (26), la lumière d'au moins un moyen d'éclairage (27) étant intégrée dans l'élément conducteur de lumière (26) au niveau de la face frontale arrière et/ou de la circonférence du cylindre et sortant de l'élément conducteur de lumière au niveau de la face frontale avant.

14. Dispositif de mesure de réfraction selon la revendication 13,
**caractérisé en ce que** plusieurs diodes électroluminescentes (27) réparties sur la circonférence de l'élément conducteur de lumière cylindrique circulaire (26) servent de moyen d'éclairage.

15. Dispositif de mesure de réfraction selon une des revendications 1 à 14,
**caractérisé en ce que**,
dans le dispositif optique de projection (03) du dispositif de mesure de réfraction (01), il est prévu une source de lumière infrarouge (08) pour générer le modèle d'éclairage.

16. Dispositif de mesure de réfraction selon une des revendications 1 à 15,
**caractérisé en ce que**,
dans le dispositif optique d'observation (04) du dispositif de mesure de réfraction (01), il est prévu un écran perforé (09).

17. Dispositif de mesure de réfraction selon une des revendications 1 à 16,
**caractérisé en ce que**,
dans le dispositif optique d'observation (04) du dispositif de mesure de réfraction (01) et/ou dans le dispositif d'enregistrement Schleimpflug (17) et/ou dans le dispositif optique d'observation (24) du kératomètre, il est prévu respectivement au moins un vidéocapteur (12, 22, 29), le vidéocapteur (12, 22, 29) transmettant les données d'image sous forme d'un signal vidéo.

18. Dispositif de mesure de réfraction selon la revendication 17,
**caractérisé en ce que** les vidéocapteurs (12, 22, 29) sont réalisés à la manière d'une caméra à puce.

19. Dispositif de mesure de réfraction selon la revendication 17 ou 18,
**caractérisé en ce que** le signal vidéo est généré en format de données numérique ou est converti en format de données numérique.

20. Dispositif de mesure de réfraction selon une des revendications 1 à 19,
**caractérisé en ce que**
il est prévu comme dispositif d'exploitation du dispositif de mesure de réfraction (01) et/ou du dispositif d'enregistrement Schleimpflug (17) et/ou du dispositif d'observation (24) du kératomètre un système de traitement numérique d'images qui permet d'exploiter les données d'image numériques.

21. Dispositif de mesure de réfraction selon une des revendications 1 à 20,
**caractérisé en ce que**
le dispositif de mesure de réfraction (01) présente une caméra d'ajustement pour l'orientation dans la bonne position de l'oeil à examiner (02) pendant l'examen.

22. Dispositif de mesure de réfraction selon la revendication 21,
**caractérisé en ce que**
le dispositif optique d'observation (29) du kératomètre peut aussi être utilisé comme caméra d'ajustement et/ou comme caméra de vue d'ensemble.
